Europäisches Patentamt

European Patent Office

Office européen des brevets

(19)

(11) Publication number: **0 048 473**

A1

(12) **EUROPEAN PATENT APPLICATION**

(21) Application number: **81107431.9**

(22) Date of filing: **18.09.81**

(51) Int. Cl.³: **A 61 K 33/30**
**A 61 K 31/195**
**//(A61K33/30, 31/195, 31/19)**

(30) Priority: **19.09.80 IL 61080**

(43) Date of publication of application:
**31.03.82 Bulletin 82/13**

(84) Designated Contracting States:
**AT BE CH DE FR GB IT LI LU NL SE**

(71) Applicant: Garfinkel, Doron, Dr.
Tarmab Street 32
Rishon Lezion(IL)

(71) Applicant: Madjar, Chaim
Israel Shachar Street 14
Rehovot(IL)

(72) Inventor: Garfinkel, Doron, Dr.
Tarmab Street 32
Rishon Lezion(IL)

(72) Inventor: Madjar, Chaim
Israel Shachar Street 14
Rehovot(IL)

(74) Representative: Weickmann, Heinrich, Dipl.-Ing
Patentanwälte Dipl.Ing.H.Weickmann et al,
Dipl.Phys.Dr.K.Fincke Dipl.Ing.F.A.Weickmann
Dipl.Chem.B.Huber, Dr.-Ing.H.Liska Möhlstrasse 22
D-8000 München 86(DE)

(54) A novel pharmaceutical composition containing zinc salts.

(57) A novel pharmaceutical composition containing zinc salts associated with L-glutamic acid and if desired, other non-toxic L-amino acids. The pharmaceutical formulations and compositions of this invention has been found to be effective in combatting and healing a large spectrum of diseases, some of which are known to be caused by zinc deficiency as well as others which have hitherto not been known to be associated with zinc-deficiency.

EP 0 048 473 A1

## A novel pharmaceutical composition containing zinc salts

This invention relates to novel pharmaceutical compositions containing zinc ions, which are suitable for administration to living cells in general and in particular to man. The pharmaceutical formulations and compositions of the invention have been found to be effective in combatting and healing a large spectrum of diseases as will be listed hereinafter in detail.

## Background of the invention

It has been known for some time that zinc is an essential element in plants, animals and man (Halstead S.A. et al; J. Nutr. 104, p.347, 1974). Spontaneous or experimentally induced zinc deficiency in animals produces growth retardation, hypogonadism, anorexia, alopecia, diarrhea and characteristic dermatitis (Shanklin S.H. et al; J. Nutr. 96, p. 101, 1968).

In man, acrodermatitis enteropathica (AE) is an inborn error in zinc absorption, which leads to central nervous system disease, gastrointestinal malfunction, characteristic skin lesions, and severe immuno-deficiency (Danbolt N, Acta Dermato-Venereol, 36, p. 257, 1956; Moynahan E.J. Lancet I, 399, 1974; Neldner K.H. et al, N. Engl. J. Med. 292, 879, 1975; Caggiano V. et al, Am. J. Med. Sei. 257, 305, 1979; Rodin A.E. et al, Emer. J. Clin. Phathol. 51, 315, 1969 and Julius, R. et al, J. Pediatr. 83, 1007, 1973).

The laboratory criteria for the diagnosis of zinc deficiency are not completely well established. The response to therapy with zinc is probably the most reliable index for making a diagnosis of a zinc deficient state in man (Zinc and Copper in Clinical Medicine, SP Medical and Scientific Books, 1978, p. 8--hereinafter "Zinc and Copper").

An adult human body contains approximately 2g of zinc. Liver, kidney, bone, retina, prostate and muscle appear to be rich in zinc. In the plasma, most of the zinc present is protein-bound (Prasad, Oberleas, 1970, J. Lab. Clin. Med. 76, 416). Serum albumin binds most zinc.

A small fraction of plasma zinc is bound to amino acids particularly to histidine, glutamine, threonine, cystine and lysine, which showed the most marked effects with respect to zinc binding. It is suggested that the amino acid bound fraction of zinc may have an important role in biological zinc transport of this element (Zinc and Copper, pp. 9-10). Many enzymes need zinc for their function. At least 30 enzymes have been identified to require zinc for their activity. If different enzymes from different species are included, then over 70 zinc metallo-enzymes are now on record (Vallee B.L. et al, 1957 N. Engl. J. Med. 257, 1055).

Zinc has been found in both DNA and RNA polymerase. The activity of RNase is inhibited by the presence of zinc, thus zinc seems to play a very significant and important role in RNA/DNA synthesis and catabolism of RNA. Growth retardation so commonly seen as a result of zinc deficiency is most likely due to its adverse effect on nucleic acid metabolism and decreased protein synthesis.

It has also now been shown that the activity of various zincdependent enzymes is affected adversely as a result of zinc deficiency. Changes in alkaline phosphatase, alcohol dehydrogenase, carboxy-peptidase and carbonic anhydrase have been reported in tissues of experimental animals (Zinc and Copper, p. 9).

The correction of symptoms due to or related to zinc deficiency in man has also been reported in the literature. 15 to 30 mg of zinc were administered orally per diem, provided the diet contained sufficient animal protein. Zinc sulfate, zinc acetate or zinc gluconate were used as sources of zinc. However, amounts up to 110 mg of zinc per day have been administered for wound healing and to sickle cell anemia patients.

The margin of safety in the therapeutic use of zinc is wide. In fact 1g or more can be administered before toxic manifestations are observed (loc. cit. pp. 11 and 12).

Zinc metabolism in humans after oral and intravenous administration of zinc has also been studied (Aamoydt R.L. et al, the American Journal of Clinical Nutrition 32, March 1979, pp. 557-569).

Blood and serum from cancer patients generally show subnormal zinc levels. Return to normal levels was usually associated with favorable prognosis (Addink N.W.H. et al, Cancer 12, 1959, pp. 544-551). However, their conventional treatment by chemotherapy and irradiation was found to increase the amount of zinc excreted in urine, thus in effect lowering the zinc levels.

A large amount of literature has also been published recently relating to etiological factors responsible for zinc deficiency in man as in laboratory animals. They can be summed under the headings: Nutritional  Factors, Liver Disease, Malabsorption, Renal Diseases, Burns, Pregnancy and others, which are still being investigated (Zinc and Copper, pp. 3-4). Genetic disorders, particularly sickle cell anemia, acrodermatitis enteropathica, anorexia nervosa and iatrogenic causes have also been found to be associated with manifestations of zinc deficiency (loc. cit., p. 5).

Investigations into the incorporation of non-essential amini acids into skin proteins of rats showed that the incorporation was significantly reduced in zinc-deficient rats as compared with zinc supplemented pairfed rats. The most striking difference was the 70% decrease in the specific activity of skin protein in the zinc-deficient rats after glycine-3-$^{14}$C injection. The incorporation of L-proline-U-$^{14}$C and L-cystine-3-$^{14}$C was reduced to less than 45% of the normal value. Zinc deficient rats also incorporated 30% less L-glutamic acid-U$^{14}$C into skin protein than did the zinc supplemented controls. Similar results were obtained when the incorporation of essential amino acids was tested.

The findings of reduced incorporation of glycine and proline, two major precursors of collagen, into zinc deficient rats indicate that zinc may be involved in collagen synthesis. Results show that zinc deficient rats incorporated significantly less labeled glycine and proline into soluble fractions of skin collagens.

The investigators arrived at the conclusion that the above reported findings may offer some mechanism why zinc therapy led to complete clearance of skin lesions in acrodermatitis enterophatica, as reported by Moynahan, 1974, Lancet 2, 399-400 (Zinc and Copper, pp. 30-33).

Zinc deficiency has also been experimentally produced in laboratory animals and in man and resulted in some of the clinical features described above. These features were promptly reversed following administration of zinc. The studies also suggested that zinc is needed for normal maintenance of blood ammonia level, cholesterol and triglycerides (Zinc and Copper, p. 11).

Notwithstanding all the above findings, therapy of humans by administration of zinc compounds together with glutamic acid, glycine or proline has not been suggested nor investigated hitherto.

## The Invention

Researchs carried out by applicants have led to the surprising discovery that ageing in living bodies is associated with progressive zinc-deficiency manifestations.

Other investigations to find ways to enlarge the spectrum of clinical features which can be ameliorated by zinc therapy as well as to find pharmaceutical zinc compositions of increased efficacy compared to that achieved hitherto by zinc therapy led to the surprising discovery that mixtures of certain zinc salts together with certain amino acids are much more effective than the zinc compositions and formulations used hitherto.

The invention thus relates to a novel pharmaceutical composition constituted of one or more zinc salts, the anions of which are selected from one or more members of the group including sulfate, carbonate and phosphate and/or non-toxic organic anions, said zinc salts being associated with L-glutamic acid, in a proportion, such that one part by wt. of zinc is admixed with 0,5 to 1,5 parts by wt. of L-glutamic acid.

The composition preferably contains in addition other amino acids selected from one or more members of the group constituted of glycine, L-proline, L-leucine, L-arginine and L-threonine. The addition of vitamin $B_6$ (pyridoxine) has also proved to be beneficial.

Zinc sulfate heptahydrate is the preferred zinc salt. When glycine is one of the ingredients of the formulation, anhydrous zinc sulfate is preferably added. The preferred organic anions are acetate, gluconate, lactate, citrate and glutamate.

L-Glutamic acid is an essential ingredient of the inventive composition as indicated above. The addition of proportions of glutamic acid in escess of the maximal proportion defined above, does not improve the therapeutic efficacy of the composition since excess glutamic acid is used by the body in other cycles.

Therapeutically effective does of zinc range from 20 mg/ diem to 200 mg/diem. There again, excess amounts of zinc do not improve the efficacy of the composition since they are excreted with urine.

A therapeutic composition containing a zinc salt and L-glutamic acid in the proportions stated above, together with minor proportions of glycine and/or L-arginine have proved to be particularly effective.

Although the importance of amino acid-bound fractions of zinc in the biological transport of zinc has been reported in the literature (vide supra) all the zinc comporsitions according to the invention are novel.

Clinical trials carried out by applicants established the fact that a substantial number of diseases associated
with zinc deficiency phenomena or with malfunction of zinc
metabolixm, which have hitherto not responded to conventional zinc therapy could be alleviated and in many cases
completely healed by oral and intravenous administration
of the composition of the invention.

Moreover, the applicants also discovered the surprising
fact that the composition according to the invention is
also effective as treatment of a number of diseases the
connection of which with zinc deficiency manifestations
was neither suspected nor observed and the treatment of
which with zinc was therefore not suggested. In fact, the
applicants have established that. the inventive composition
brings about the optimal functioning of all living cells,
even in generally healthy persons. In other words the
composition may be said to act as a vitamin.

Thus applicants have established that the inventive compositions are effective:

1. In increasing the life expectancy of man by slowing
   down or arresting the ageing process of the body tissues.

2. In causing a feeling of well-being.

3. In speeding up the rate of the healing process in case
   of physical tissue damage as a result of pressure,
   temperature, electric shock, radiation, etc. or due to
   the administration of toxic or carcinogenic substances.

4. Arteries: In retarding or arresting and finally healing
   damage to blood vessels due to arterio sclerosis;

   a) in general, it corrects states of hypertension by a process comprising regeneration of the blood vessels in the affected region;

b) in coronary arteries, it alleviates pain in cases of coronary insufficiency (anginal syndrome);

c) in cerebral arteries, it prevents malfunction of the brain due to cerebrovascular ischemia, it improves the blood supply to the eyes and prevents blindness resulting from insufficient supply;

d) in peripheral arteries, in the extremities, it alleviates and stops pain due to intermittent claudication; it corrects trophic changes of the skin and/or its appendages, i.e. hair follicles, sweat glands and sebum;

e) quick healing of skin-ulcers (e.g. ulcus cruris);

f) parenchycatic organs: it improves the blood flow to these organs and restores their normal function.

The above mentioned effects of the treatment apply also in the presence of systemic and/or metabolic diseases (e.g. diabetes, hyperlipidemia) including malfunction of the internally secretory glands.

5. Veins:

a) it alleviates pain caused by varicose veins in the legs and subsequently it reduces the affected varicose veins and new veins are formed (prevention of venous stasis);

b) it heals chronic venous insufficiency.

6. Skin:

    a) it prevents degenerative and ageing changes in skin and its appendages such as the appearance of wrinkles, shedding and drying up of hair;

    b) it accelerates the appearance of new granulation tissue in the case of wounds, ulcers or following skin surgery, and thus accelerates the healing process;

    c) it causes definitive regeneration of atrophic skin, including new hair-growth on bald patches (due to any cause) as well as new sweat glands and sebum;

    d) it heals basal cell carcinoma.

7. Heart:

    a) it heals the diseases mentioned in paragraph 4b;

    b) it has a positive inotropic effect: increases the cardiac output by increasing cardiac muscle contraction;

    c) it prevents arrhytmia of any kind;

    d) it reduces the size of nexrotic tissue after myocardial infarction.

8. Liver: It improves the functioning of liver cells with regard to metabolism of the main constituents of food, the formation of bone, the formation of clotting agents, bile and other substances needed for the detoxification of toxic chemical substances (e.g. the cells become more resistant to damage due to alcohol).

9. Intestines: It heals various inflammations of the intestines including Crohn's disease and colitis ulcerosa of the small intestine.

10. Eyes:

   a) it improves vision generally, particularly of old people by improving the blood supply; it has also a beneficial effect on the retinal cells;

   b) it heals cataracts and other clinical features which affect the proper transparency of the vatious parts of the eye (cornea, chambers, lense, and vitreous body);

   c) it heals senile macular degeneration.

11. Muscles, joints and connective tissues:

   a) it increases muscular strength (Myasthenia Gravis);

   b) it improves the rate of healing in cases of physical or other damage (vide paragraph 3 supra);

   c) it alleviates arthritic pains.

12. a) it heals inborn errors of metabolism;

   b) it heals hereditary states in the field of hematology such as sickle cell anemia;

   c) it improves the condition of children and adults who suffer from Cystic Fibrosis, Hypogonadism, Dwarfism, Mongoloidism, etc.

The administration of the inventive composition to patients who are treated by diuretics, cytotoxic agents or irradiation is absolutely essential.

Investigations of the effect of the inventive composition on diseases such as influenza, common cold, heart-muscle malfunction, cerebro-vascular ischemia, cerebro-vascular accident (CVS), Burger's disease, gangrene, on muscular function, on diseases of the kidneys and the lungs, on the nervous system, on gout and pseudo gout, on diabetes mellitus, on connective tissue diseases, on endocrinological and urological diseases, and in connection with psychotherapy and menometrorrhagia, all of which are not known to be caused by, or connected with, zinc deficiency or faulty cell plasma zinc metabolism, are being carried out by applicants and have so far shown promising results. The investigations are being continued.

Administration and Dosage

The composition according to the invention is being administered at present in the form of capsules or enteric coated tablets containing 400 mg of composition. A number of formulations will be given by way of example only:

Formulation A: <u>Zinc sulfate - L-glutamic acid mixture</u>

| | |
|---|---|
| L-glutamic acid | 50 - 70 mg |
| zinc sulfate $7H_2O$ | 330 - 350 mg |

Formulation B: <u>Zinc sulfate - amino acids - vitamin $B_6$ mixture</u>

| | |
|---|---|
| L-glutamic acid | 82 mg |
| glycine | 48 mg |
| L-proline | 27 mg |
| L-leucine | 14 mg |
| L-arginine | 7 mg |
| L-threonine | 3 mg |

Formulation B: <u>Zinc sulfate - amino acids - vitamin B$_6$ mixture</u>

| | |
|---|---|
| Vitamin B$_6$ | 27 mg |
| zinc sulfate | 192 mg |

Formulation C: <u>Zinc sulfate - L-glutamic acid-glycine mixture</u>

| | |
|---|---|
| L-glutamic acid | 90 mg |
| glycine | 52 mg |
| zinc sulfate | 258 mg |

Formulation D: <u>Zinc sulfate -L-glutamic acid-L-arginine mixture</u>

| | |
|---|---|
| L-glutamic acid | 85 mg |
| glycine | 50 mg |
| L-arginine | 50 mg |
| zinc sulfate | 215 mg |

The whole or part of the zinc sulfate ingredient may be substituted by equivalent quantities of zinc carbonate, zinc phosphate, zinc acetate, zinc gluconate, zinc lactate and zinc citrate.

The normal dosage administered to adult humans is one capsule or tablet a day, but up to four capsules a day have been administered without causing toxic side-effects.

The various ingredients of the formulations are admixed in powder form and then filled into capsules or tabletted. The composition has also been formulated for administration to volunteers by the intravenous route. The results obtained have been definitely promising.

<u>Case Histories</u>

<u>Case 1</u>

Patient: Woman aged 56

Diagnosis:

a) Mitral Stenosis and Cardiomyopathy, Atrial Fibrillation, severe coronary insufficiency. Severe congestive heart failure (grade 4). Patient sleeps at night on four to five pillows.

b) State post recurrent cerebro vascular accidents (CVA). Two cerebral strokes resulting in transient hemiparesis and constant left facial nerve paresis.

c) Peripheral vascular disease. No pulse from the femoral artery and below. Legs are cold and white all the time. Veins and capillaries are enlarged. Severe intermittent claudication. Can walk only 4-5 steps at a time.

d) Anginal syndrome. Pain in the chest, particularly after strain several times a day and after walking a few steps.

e) Disturbed vision, patient cannot see her legs clearly and particularly no small items.

f) Basal cell carcinoma on left eyebrow. Deep wound through which the bone is visible and intermittent bleeding, for more than two years. Wound does not heal. Constant falling out of hair for several years. Cannot look after herself. Her daughter has to cook for her and to dress her. Cannot perform simple chores at home. Treated by massive doses of diuretics, potassium salts and coumadin.

Treatment prescribed: One 400 mg capsule of the inventive composition a day.

After one month: Vast improvement with regard to the pain in the legs and in the chest. Able to walk distances and slopes which she was unable to walk for many years. Pain in chest recurs less often and is less severe. Vision improved. Can see her legs clearly and thread a needle. Wound in eyebrow begins to heal. Intermittent bleeding continues.

After two months: Feels well. The legs are warm. Walks quickly. Does all her chores at home. Able to walk up steps, uses public transport. Able to lift up her grandson aged 5, which she had never been able to do earlier. The wound in eyebrow has changed, now skin grows from the base of the wound.

After four months: Gneral improvement continues. No more falling out of hair. No further signs of coronary insufficiency. Sleeps on one pillow. Practically no more pain in the chest. No pain in the legs.

After 16 months: Feels well. Wound closed entirely. It seems as if hair was beginning to grow on the wound.

## Case 2

Patient: Man aged 66. Suffers from asthma. Patient has been nearly blind for 1 1/2 years.

Diagnosis: Senile macular degeneration in both eyes. Has also cataract in left eye. During eye-examination sees only less than three meters; could not count fingers from two meters away. Unable to read.

Treatment: One 400 mg capsule a day.

After one month: General condition improved. More active. Slight improvement of vision.

After two months: Vision improved. Able to play cards.

After three months: Patient reports considerable improvement. Can see for a distance of 70 m. Patient had been asked to undergo eye-surgery; has now been told that surgery was no longer indicated. According to his wife: General physical improvement and improvement of mood; previously patient had no wish to live any longer.

## Case 3

Patient: Man aged 49. Suffered myocardial infarction twice. Coronary bypass surgery in 1972 and 1975. Also resuscitation applied. Eight months prior to treatment with inventive composition, recurrence of pains in the chest. Catheterization showed thrombosis of diagonal branch of LAD (left anterior descending artery). Had been treated with Deralin, Trasicor and subsequently with Adalat. Is able to walk up to 10-12 stairs or to walk 70 m on a slight slope before pain appears. Takes 7-10 nitroglycerine tablets a day.

Treatment: One 400 mg capsule a day.

After two months: Stopped to take drugs without asking his physician. After three-four weeks took only two tablets of nitroglycerine a day or no tablet at all.

After four months: Feels excellent. Takes practically no nitroglycerine.

## Case 4

Patient: Man aged 74. Has been suffering from diabetes for 16 years. Large wound on left thigh (ulcus cruris). In 1978 a skin transplant was attempted which was rejected. Subsequently wound became infected several times. Again . pain in the leg and fever. A suppurating wound was found on the opposite side of the leg. On the X-ray a focus of osteomyelitis was seen and bacterium staphylococcus aureus grew in the wound. Following treatment with antibiotics, the temperature dropped and the discharge from the wound decreased. It was decided to treat with inventive composition.

Treatment: two capsules (400 mg) a day.

After one month: Definite improvement of the wound, which began to heal. The hole at the lateral side began to close up and the ulcer was less deep and much narrower.

After two months: Feels well. The wound on the lateral side was closed entirely. New skin grew on the central part of the ulcer. An ulcer of an area 1/4 of the original one remained.

## Case 5

Patient: Man aged 70.

Diagnosis: Diabetes mellitus treated with Daonil. CVA eight months earlier, with paralysis of half of the body from which patient had nearly recovered. There remained a difficulty to speak, facial nerve paresis and weakness in the legs; had been practically unable to walk for two years. Tires quickly, unable to climb stairs, used a walking stick occasionally. Was treated with Vasolen, Cardoxin, Rodenal.

Treatmant: One capsule (400 mg) a day.

After one month: Is more active and moves freely.

After three months: Sits down and stands up easily. Walks up to 1/2 km. Returned to work. Speaks more freely.

After four months: Further improvements in walking, does not lose equilibrium. Works and functions well.

Case 6

Patient: Man aged 53.

Diagnosis: Severe hypertension for several years, treated by 4 tablets of Deralin and Kaluril. Occasional hypertension remains. From the beginning of the treatment with inventive composition feels well. No substantial rise of blood pressure.

After six months: Takes only two Deralin tablets a day. Blood pressure 125/80.

After 14 months: Blood pressure normal. Takes one Deralin tablet per day.

After 18 months: Blood pressure normal. Deralin treatment no longer necessary. Takes one tablet of Kaluril twice a week.

## Case 7

Patient: Man aged 50.

Diagnosis: Severe Rheumatoid Arthritis for 13 years; marked ankylosis and deformation of the knee joints, elbows and proximal interphalangeal joints marked ulnar deviation; subcutaneous nodules. Decreased muscular strength, especially in the hands. Has been treated unsuccessfully with many anti-inflammatory and anti-rheumatic drugs.

Treatment: One capsule (400 mg) a day.

After several months: Patient felt increased physical power in the hands and a feeling of well-being. The joint movements seemed less painful.

After one year: Marked improvement of hand grip; can easily pull out button on the dash board of his car with two fingers--a task he was unable to do with both his hands a year earlier. Reduction in the size of the subcutaneous nodules.

## Case 8

Patient: Woman aged 50.

Diagnosis: Peripheral vascular disease; intermittent claudications; menometrorrhagia; myoma of uterus; refferred for hysterectomy.

Treatment: One capsule (400 mg) a day.

After two months: Markes improvement in walking, no sign of intermittent claudications. The vaginal bleeding decreased and almost stopped. Before taking out the uterus her gynecologist noticed a marked reduction in the size of the myoma. Exdellent post-operative recovery with quick wound healing.

<u>C l a i m s</u>

1. A pharmaceutical composition containing a zinc salt, the anion of which is selected from one or more members of the group including sulfate, carbonate, phosphate and/or non-toxic organic anions said zinc salts being associated with L-glutamic acid, in a proportion, such that one part by wt. of zinc is admixed with 0,5 to 1,5 parts by wt. of L-glutamic acid.

2. A pharmaceutical composition as claimed in claim 1 containing additionally minor proportions of one or more amino acids selected from the group comprising glycine, L-leucine, L-arginine, L-threonine and L-proline.

3. A pharmaceutical composition as claimed in claim 1, containing in addition glycine and/or L-arginine.

4. A pharmaceutical composition as claimed in any one of the preceding claims containing in addition a minor proportion of vitamin $B_6$.

5. A pharmaceutical composition as claimed in any one of the preceding claims wherein the zinc salt is zinc sulfate heptahydrate.

6. A pharmaceutical composition as claimed in claim 1, wherein the zinc salt is anhydrous zinc sulfate.

7. A pharmaceutical composition as claimed in any one of the preceding claims 1 to 4, wherein the non-toxic organic anions are selcted from the group constituted of acetate, gluconate, lactate and citrate.

8. A pharmaceutical composition as claimed in any one of the preceding claims for oral administration in the form of a capsule, each containing a therapeutically effective quantity of a zinc salt together with 0,5 to 1,5 times said quantity of L-glutamic acid.

9. A pharmaceutical composition as claimed in any one of the preceding claims 1 to 7 for oral administration in the form of an enteric coated tablet, each filled tablet containing a therapeutically effective quantity of a zinc salt together with 0,5 to 1,5 times said quantity of L-glutamic acid.

10. A pharmaceutical composition as claimed in any one of claims 1 to 7 formulated for intravenous administration.

11. A pharmaceutical composition containing a zinc salt and L-glutamic acid for oral administration in unit-dosage form, substantially as hereinbefore exemplified in any one of the formulations.

0048473

European Patent Office

**EUROPEAN SEARCH REPORT**

Application number

EP 81 10 7431

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| X | <u>FR - A - 2 269 352</u> (AB DRACO)<br>* Page 6, lines 1-21; claims 1-4 *<br>& GB - A - 1 498 832<br>& US - A - 4 292 324<br><br>-- | 1-11 | A 61 K 33/30<br>31/195//<br>(A 61 K 33/30<br>31/195<br>31/19) |
| X | <u>US - A - 4 167 564</u> (NED L. JENSEN)<br>* Column 9, lines 1-46; claims 1-7 *<br><br>-- | 1-11 | |
| X | DICTIONNAIRE VIDAL, 1961, Office de Vulgarisation Pharmaceutique; Paris, FR<br>"Plurimétavit Allard",<br>"Plurimétavit Specia"; page 1311.<br>* Abstracts *<br><br>-- | 1-11 | TECHNICAL FIELDS SEARCHED (Int. Cl.³)<br><br>A 61 K 33/30<br>37/18<br>31/195<br>31/315 |
| | UNLISTED DRUGS, (card-file), September 1978<br>Chatham, N.J. US<br>"Bioscalin"<br>* Whole abstract *<br><br>-- | 1-11 | |
| P | <u>US - A - 4 225 614</u> (ANDERS E. HANSSON)<br>* Column 6, lines 15-33; claims 1,2 *<br><br>------ | 1-11 | CATEGORY OF CITED DOCUMENTS<br><br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br><br>&: member of the same patent family, corresponding document |

| The present search report has been drawn up for all claims | | |
|---|---|---|
| Place of search<br>The Hague | Date of completion of the search<br>03-12-1981 | Examiner<br>BRINKMANN |

EPO Form 1503.1 06.78